# EUROPEAN PATENT APPLICATION

(11) **EP 4 585 698 A2**
(43) Date of publication of application: **16.07.2025**
(21) Application number: 24223119.9
(22) Date of filing: 23.12.2024
(51) Int. Cl.: C12Q 1/6844

(54) **USE OF TAQNEQSSB POLYMERASE TO NORMALIZE PROBE AND PRIMER ANNEALING TEMPERATURE IN THE SINGLE-NUCLEOTIDE SNP MUTATION DETECTION REACTION**

(30) Priority: 23.12.2023 PL 44727823
(71) Applicant: Instytut Biotechnologii I Medycyny Molekularnej, 80-180 Gdansk (PL); Geneme Molecular Sp. z o.o., 80-180 Gdansk (PL)
(72) Inventor: NIDZWORSKI, Dawid, 80-180 Gda sk (PL); ZOLEDOWSKA, Sabina, 80-180 Gdansk (PL); SZEMIAKO, Kasjan, 80-287 Gdansk (PL); SKWARECKA, Marta, 80-180 Gdansk (PL)
(74) Representative: Matyka, Malgorzata

(57) **Abstract**

The subject of the present invention is one application of TaqNeqSSB polymerase in normalizing the temperature of attachment of probes and primers of multiple independent amplification reactions in order to standardize the method of identifying multiple single-nucleotide mutations (SNPs) and to be able to carry out multiple independent assays at the same time and under the same temperature-time profile.

## Description

The subject of the present invention is one application of TaqNeqSSB polymerase in normalizing the temperature of attachment of probes and primers of multiple independent amplification reactions in order to standardize the method of identifying multiple single-nucleotide mutations (SNPs) and to be able to carry out multiple independent assays at the same time and under the same temperature-time profile.

DNA polymerase plays a key role in DNA replication and repair. This enzyme is used in the Polymerase Chain Reaction (PCR), where it catalyzes DNA synthesis *in vitro* by attaching nucleotides to the 3'OH end of the DNA strand. DNA polymerases show variation in terms of speed of catalysis, processivity, presence or absence of interacting protein subunits, and display of nucleolytic activity. A general division of DNA polymerases classifies them into 7 families: A, B, C, D, X, Y and RT. Bacterial DNA polymerases are represented in families A, B, C, X, Y, archenic ones in B, D, X and Y, and eukaryotic DNA polymerases in families A, B, X, Y and RT. This diverse division takes into account the differences between organisms, as well as the specific characteristics of these enzymes. The structure of these polymerases, despite significant differences, shows similarity in terms of the presence of three basic subdomains: finger, metacarpal and thumb, which are assigned well-defined functions.

The most widely used DNA polymerase of bacterial origin is Taq polymerase, isolated from Thermus aquaticus. This enzyme, discovered in 1976, revolutionized the field of molecular biology. It consists of 832 amino acid residues with a molecular mass of 94 kDa, and achieves its highest activity at temperatures between 72°C and 80°C. The polymerization process is made possible by the attachment of DNA to the enzyme's active center, where amino acid residues such as Arg682, Lys785, Tyr766, Arg821 and His811 play a key role. Taq DNA polymerase has three domains: an exonucleolytic 5'→3', an inactive exonucleolytic 3'→5' and a polymerizable 5'→3'. Removal of the exonucleolytic domain leads to a functional protein, but with partially altered characteristics compared to the native enzyme. Deprived of the 5'→3' exonuclease activity of DNA polymerase TaqΔ289 (also known as TaqStoffel, KlenTaq) is characterized by increased thermostability, a slightly higher requirement for Mg²⁺ ions, and the newly synthesized DNA strand is endowed with fewer errors.

Modern research is focused on finding new DNA polymerases or improving existing ones to meet the growing demands of amplifying diverse DNA matrices. The discovery of Taq polymerase in fusion with the NeqSSB protein that binds all types of nucleic acids has led to improvements in its functional properties, mainly an increase in affinity for the DNA matrix, resulting in improved characteristics such as processivity, efficiency, specificity or the ability to amplify difficult DNA matrices.

NeqSSB protein belongs to the SSB-like protein family. SSB (Single Stranded DNA Binding Protein) proteins are present in all forms of life. They take part in key processes such as DNA replication, recombination and repair, where fragments of single-stranded DNA are formed. They have a protective function, protecting single-stranded DNA (ssDNA) from degradation, while interacting with other cellular proteins. The protein's binding mechanism to ssDNA is based on the packing of aromatic amino acid residues between bases in the oligonucleotide chain and the interaction of positively charged amino acid residues with the phosphate backbone of ssDNA. There are also SSB-like proteins, such as NeqSSB, which differ in their properties from classical SSB proteins. They are synthesized by mammalian, yeast, archaeon and bacterial cells. They differ in molecular weight, number of subunits and binding site, depending on the source organism. The NeqSSB protein is derived from the hyperthermophilic archaeon *Nanoarchaeum equitans*, parasitizing the craenarchaeon *Ignicoccus hospitalis. Nanoarchaeum equitans* has the smallest known genome, but a full set of enzymes involved in DNA replication, repair and recombination, including the SSB protein. The NeqSSB protein, like others in this family, has the ability to bind DNA naturally. It consists of 243 amino acid residues and has a single OB domain. It exhibits biological activity as a monomer, similar to some viral SSB proteins. Atypically, NeqSSB binds to various forms of DNA (ssDNA, dsDNA) and mRNA, without structural preference. Domains I, II and III are responsible for binding to nucleotide acids, with domains II and III having the highest binding strength. The protein itself is also highly thermostable, with a half-life at 100°C of 5 minutes and a melting point of 100.2°C.

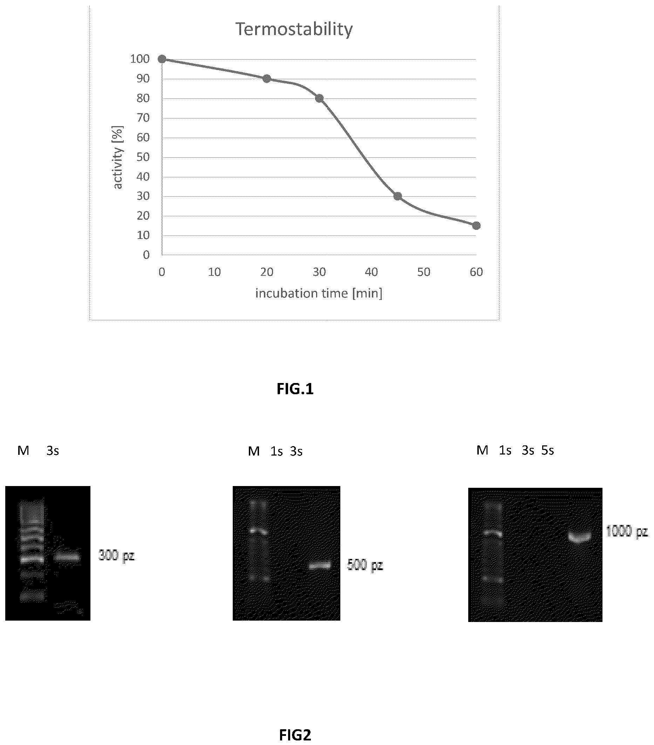

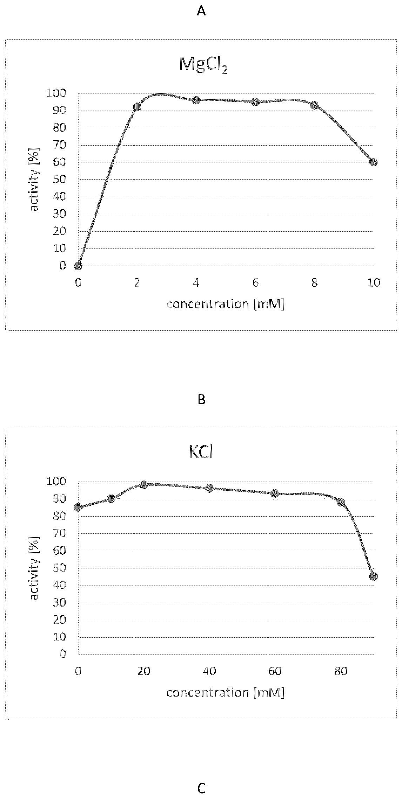

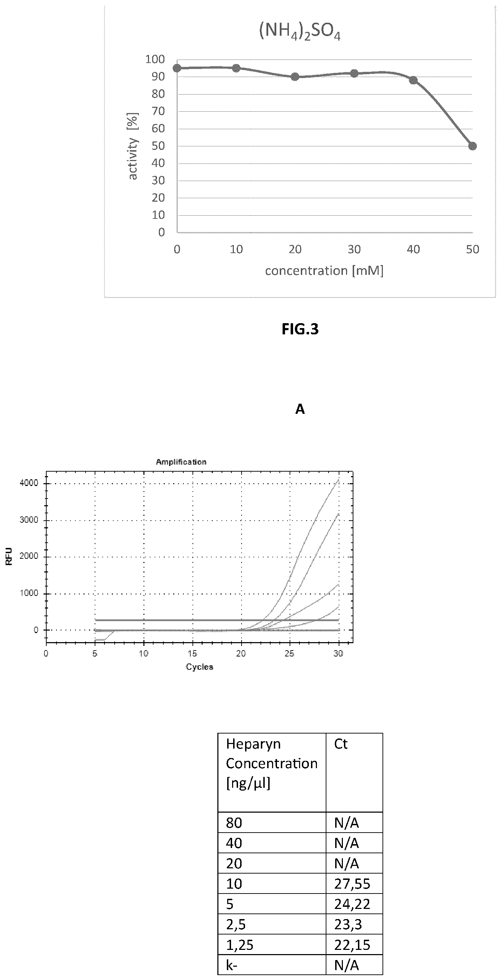

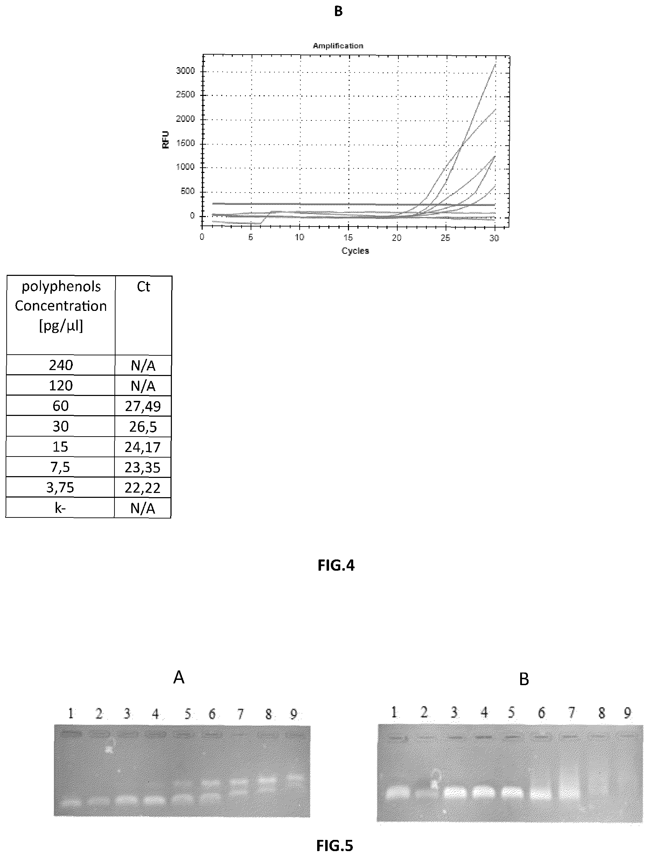

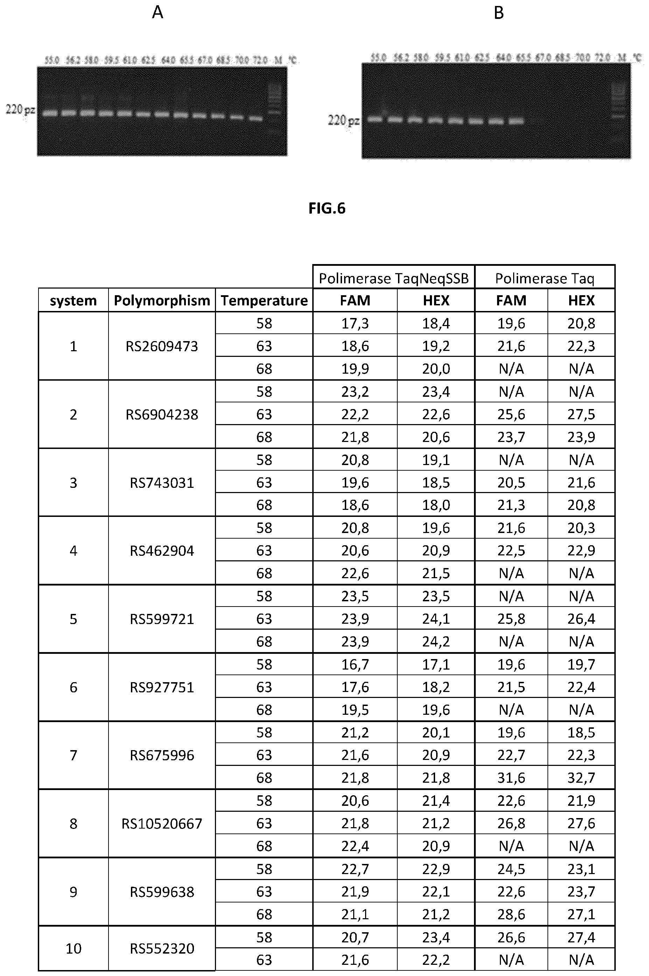

TaqNeqSSB polymerase, thanks to its fusion with a DNA-binding protein, has improved thermostability (up to 40 minutes at 95° C, FIG1) and thus enhanced ability to amplify difficult matrices. In addition, the presence of fusion improves the processivity and speed of DNA synthesis to a level of 1000 bp within 5 s (FIG2). The polymerase works efficiently over a large cofactor range of 2 - 8 mM MgCl₂ (FIG3A) and tolerates high concentrations of KCl salt (up to 80 mM, FIG3B) and (NH₄)₂SO₄ (up to 37.5 mM, FIG3C) making it much easier and faster to optimize the PCR reaction. Improved by the presence of fusion, the sensitivity and increased affinity for DNA make the DNA template in the amplification reaction selectively "captured" from the reaction mixture and even in the presence of inhibitors can be carried out more efficiently. In the case of compounds whose inhibition involves chelation of magnesium ions from the reaction mixture, the ability of the fusion DNA polymerase to amplify efficiently at lower cofactor concentrations may explain its increased resistance to such inhibitors in the reaction mixture. TaqNeqSSB polymerase was shown to be resistant to the presence of inhibitors such as heparin and polyphenols in the reaction mixture at levels of 10 ng/ul and 60 pg/ul, respectively (FIG4). The improved characteristics of the TaqNeqSSB DNA fusion polymerase compared to non-fusion polymerases are most likely due to differences in their affinity and mode of binding to DNA. This is indicated by the results obtained in the DNA binding assay (FIG5). The presence of an additional structure with the ability to bind to both ssDNA and dsDNA increased the affinity of Taq DNA polymerase for both types of nucleic acids. This is also confirmed by literature studies of DNA binding by DNA polymerase and other DNA-binding proteins, i.e. SSB proteins, which indicate that SSB proteins have a higher affinity for DNA than for DNA polymerases for DNA. Moreover, DNA binding by Taq DNA polymerase occurs in a completely different way than DNA binding by fusion DNA polymerases. As Olszewski and colleagues point out, in fusion DNA polymerase it is the protein that is responsible for such specific and preferential DNA binding, as suggested by the protein-DNA complexes formed and visible in the gel that are characteristic of SSB proteins . The results of the EMSA assay indicate that TaqNeqSSB DNA polymerase exhibits higher affinity for single-stranded as well as double-stranded DNA compared to the reference Taq DNA polymerase (FIG6). The results indicate that the fusion DNA polymerase is capable of performing PCR reactions even at 72°C, while the optimal primer attachment temperature was 55°C. In the case of the reference Taq DNA polymerase, a PCR product was obtained at the maximum primer attachment temperature of 65.5°C. The results may indicate the enhanced ability of the TaqNeqSSB fusion DNA polymerase to stabilize the template-starter complex compared to Taq DNA polymerase. The presence of an additional DNA-binding protein can stabilize the single-stranded DNA template or double-stranded complex formed by hybridization, and the PCR reaction can occur at a higher primer attachment temperature than with the reference DNA polymerase. This stabilization is likely why a PCR reaction carried out with a fusion DNA polymerase can occur with higher specificity and over a relatively large range of primer attachment temperatures. All of this leads to the effect that TaqNeqSSB fusion DNA polymerase is an ideal candidate for use in multiple single-nucleotide mutation (SNP) assay reactions and for performing multiple independent assays at a single time and under an identical temperature-time profile. Temperature normalization for multiple molecular targets and different pairs of primers and probes is possible using a polymerase with features such as those demonstrated by TaqNeqSSB.

The subject of the invention is a TaqNeqSSB fusion polymerase for use in normalizing the temperature of attachment of primers and probes of independent systems identifying several different single-nucleotide mutations in genomic DNA using the example of 12 qPCR systems with TaqMan probes identifying 12 SNP polymorphisms in human genomic DNA in a single temperature-time profile

The application of the fusion polymerase is illustrated by the normalization of systems identifying such SNP polymorphisms as RS2609473, RS6904238, RS743031, RS462904, RS599721, RS927751, RS675996, RS10520667, RS599638, RS552320, RS10418577, RS975336.

Sequences of gene fragments containing the above polymorphisms are shown in SEQ1-SEQ12, respectively
Applications where systems include:
The nucleotide sequence of forward primer RS2609473_F shown on SEQ13,
The nucleotide sequence of reverse primer RS2609473_R shown on SEQ14,
The nucleotide sequence of probe RS2609473_P_G , which has the sequence shown in SEQ15,
The nucleotide sequence of probe RS2609473_P_T, which has the sequence shown on SEQ16,
The nucleotide sequence of forward primer RS6904238_F shown on SEQ17,
The nucleotide sequence of reverse primer RS6904238_R shown on SEQ18,
The nucleotide sequence of probe RS6904238_P_T , which has the sequence shown in SEQ19,
The nucleotide sequence of probe RS6904238_P_C, which has the sequence shown on SEQ20,
The nucleotide sequence of forward primer RS743031_F shown on SEQ21,
The nucleotide sequence of reverse primer RS743031_R shown on SEQ22,
The nucleotide sequence of the probe RS743031_P_G , which has the sequence shown on SEQ23,
The nucleotide sequence of the probe RS743031_P_A , which has the sequence shown on SEQ24,
The nucleotide sequence of forward primer RS462904_F shown on SEQ25,
The nucleotide sequence of reverse primer RS462904_R shown on SEQ26,
The nucleotide sequence of probe RS462904_P_T, which has the sequence shown on SEQ27,
The nucleotide sequence of probe RS462904_P_G , which has the sequence shown on SEQ28,
The nucleotide sequence of forward primer RS599721_F shown on SEQ29,
The nucleotide sequence of reverse primer RS599721_R shown on SEQ30,
The nucleotide sequence of the RS599721_P_G probe, which has the sequence shown in SEQ31,
The nucleotide sequence of probe RS599721_P_A, which has the sequence shown in SEQ32,
The nucleotide sequence of forward primer RS927751_F shown on SEQ33,
The nucleotide sequence of reverse primer RS927751_R shown on SEQ34,
The nucleotide sequence of probe RS927751_P_G, which has the sequence shown on SEQ35,
The nucleotide sequence of probe RS927751_P_A, which has the sequence shown on SEQ36,
The nucleotide sequence of forward primer RS675996_F shown on SEQ37,
The nucleotide sequence of reverse primer RS675996_R shown on SEQ38,
The nucleotide sequence of probe RS675996_P_T, which has the sequence shown on SEQ39,
The nucleotide sequence of the RS675996_P_C probe, which has the sequence shown on SEQ40,
The nucleotide sequence of forward primer RS10520667_F shown on SEQ41,
The nucleotide sequence of reverse primer RS10520667_R shown on SEQ42,
The nucleotide sequence of probe RS10520667_P_T, which has the sequence shown on SEQ43,
The nucleotide sequence of the RS10520667_P_C probe, which has the sequence shown on SEQ44,
The nucleotide sequence of forward primer RS599638_F shown on SEQ45,
The nucleotide sequence of reverse primer RS599638_R shown on SEQ46,
The nucleotide sequence of the RS599638_P_T probe, which has the sequence shown on SEQ47,
The nucleotide sequence of the RS599638_P_C probe, which has the sequence shown on SEQ48,
The nucleotide sequence of forward primer RS552320_F shown on SEQ49,
The nucleotide sequence of reverse primer RS552320_R shown on SEQ50,
The nucleotide sequence of the RS552320_P_A probe, which has the sequence shown on SEQ51,
The nucleotide sequence of the RS552320_P_G probe, which has the sequence shown on SEQ52,
The nucleotide sequence of forward primer RS10418577_F shown on SEQ53,
The nucleotide sequence of reverse primer RS10418577_R shown on SEQ54,
The nucleotide sequence of probe RS10418577_P_A, which has the sequence shown on SEQ55,
The nucleotide sequence of probe RS10418577_P_G, which has the sequence shown on SEQ56,
The nucleotide sequence of forward primer RS975336_F shown on SEQ57,
The nucleotide sequence of reverse primer RS975336_R shown on SEQ58,
The nucleotide sequence of the RS975336_P_G probe, which has the sequence shown on SEQ59,
The nucleotide sequence of the RS975336_P_A probe, which has the sequence shown on SEQ60.

The primers and probes defined above SEQ.13-SEQ.60 have different attachment temperatures for molecular targets and are specific for the 12 SNP polymorphic sequences RS2609473, RS6904238, RS743031, RS462904, RS599721, RS927751, RS675996, RS10520667, RS599638, RS552320, RS10418577, RS975336 listed earlier.

Also the subject of the invention is a method of normalizing the temperature of attachment of primers and probes of multiple systems to a single optimal one, using the example of the polymorphisms mentioned above, involving steps:
1) DNA amplification using primers and probes with SEQ.13 - SEQ.60 in a temperature gradient
2) Detection of the formed complexes by observing the increase in fluorescence of the dye with which the probe is labeled.
3) Selection of a common temeperature for all systems at which Ct is as low as possible and similar for each system

A method where the human gene material is a swab, saliva, blood, tissue or other biological samples containing human genomic DNA.

A method where TaqNeqSSB polymerase is used in step 1).

A method where labeled probes are used in step 2).

A kit for use in the manner defined above, including:
Starters
Probes
TaqNeqSSB DNA polymerase with cofactor
Deoxyribonucleoside triphosphates
Reaction buffer

The kit contains reagents required for the amplification steps: denaturation, hybridization, DNA strand synthesis.

Primers and probes containing SEQ.13 - SEQ.60 for use in detecting and identifying single-nucleotide mutation numbers RS2609473, RS6904238, RS743031, RS462904, RS599721, RS927751, RS675996, RS10520667, RS599638, RS552320, RS10418577, RS975336.

### Description of Figures and Sequences:

**FIG.1** - shows graphs of the change in TaqNeqSSB DNA polymerase fusion activity during incubation at 95°C. Activity was determined using GelAnalyzer software based on the intensity of the resulting PCR products in an ethidium bromide agarose gel
**FIG.2** - shows electrophoretic separation in 1.5% agarose gel with ethidium bromide showing the results of PCR reactions with increasing elongation times leading to DNA fragments of 300 bp (base pairs), 500 bp and 1000 bp. PCR products were obtained using TaqNeqSSB DNA polymerase, the elongation times used in the PCR reaction are indicated above the paths, M - 100bp ThermoScientific marker
**FIG.3** - shows graphs of the effect of magnesium ion concentration MgCl₂ (A), salt concentration KCl (B), salt concentration (NH₄)₂SO₄ (C) on the efficiency of DNA amplification carried out with the fusion DNA polymerase TaqNeqSSB. Activity was determined using GelAnalyzer software based on the intensity of the PCR products obtained in an ethidium bromide agarose gel
**FIG.4** - shows a plot of the dependence of fluorescence on the duration of the qPCR amplification reaction and a tabala with Ct values for serial 2-fold dilutions of heparin (A) and polyphenols (B) added to the reaction mixture
**FIG.5** - shows the results of the DNA electrophoretic mobility delay assay in a 2% agarose gel in the presence of TaqNeqSSB (A) and Taq (B) DNA polymerases from ssDNA and dsDNA. The reaction mixture contained 10 pmol (dT₇₆) and 2.5 pmol of PCR product with a length of 100 pz, the paths numbered from 1 to 9 indicate:
   for A:
      1 - d(T)₇₆
      2 - 100 pz
      3 - d(T)₇₆+100 pz +3.3 pmol of fusion polymerase
      4 - d(T)₇₆+100 pz +6.6 pmol of TaqNeqSSB fusion polymerase
      5 - d(T)₇₆+100 pz +13.2 pmol of TaqNeqSSB fusion polymerase
      6 - d(T)₇₆+100 pz +26.4 pmol of TaqNeqSSB fusion polymerase
      7 - d(T)₇₆+100 pz +52.8 pmol of TaqNeqSSB fusion polymerase
      8 - d(T)₇₆+100 pz +105.6 pmol of TaqNeqSSB fusion polymerase
      9 - d(T)₇₆+100 pz +211.2 pmol of TaqNeqSSB fusion polymerase
   for B
      1 - d(T)₇₆
      2 - 100 pz
      3 - d(T)₇₆+100 pz +24.6 pmol of Taq DNA polymerase
      4 - d(T)₇₆+100 pz + 49.2 pmol of Taq DNA polymerase
      5 - d(T)₇₆+100 pz + 98.4 pmol of Taq DNA polymerase
      6 - d(T)₇₆+100 pz + 196.8 pmol of Taq DNA polymerase
      7 - d(T)₇₆+100 pz + 393.6 pmol of Taq DNA polymerase
      8 - d(T)₇₆+100 pz + 787.2 pmol of Taq DNA polymerase
      9 - d(T)₇₆+100 pz + 1574.4 pmol of Taq DNA polymerase
**FIG.6** - shows electrophoretic separation in 1.5% agarose gel ethidium bromide of PCR products obtained by amplification at increasing primer attachment temperatures from 55°C to 72°C using 1 U TaqNeqSSB (A) and Taq (B) DNA polymerase. The applied primer attachment temperatures in the PCR reaction are indicated above the paths, M - DNA marker HyperLadder II (DNA Gdańsk); 220 bp (base pairs).

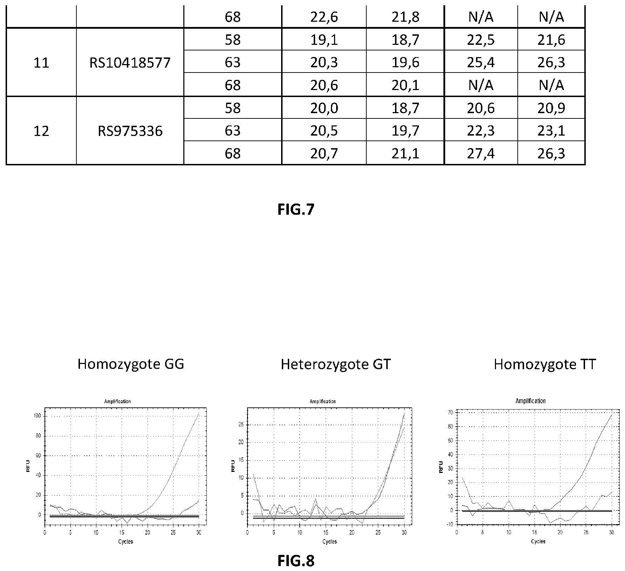

**FIG.7****-** shows a table with Ct values for systems identifying 12 SNP polymorphisms in qPCR reactions using TaqNeqSSB and Taq fusion polymerase at three different primer attachment temperatures: 58°C, 63⁽°⁾C and 68°C
**FIG.8** - shows graphs of fluorescence dependence on reaction time for identification of SNP RS2609473 polymorphism using oligomers (SEQ13-SEQ16), where the matrix was human saliva samples diluted 32-fold in saline and added directly to the qPCR reaction. Green indicates the system identifying the T allele, red the G allele.

**SEQ.1** - shows the nucleotide sequence of a fragment of human genomic DNA flanking the RS2609473 polymorphism, the letter N indicates the mutation site corresponding to the G or T nucleotide
**SEQ.2** - shows the nucleotide sequence of a fragment of human genomic DNA flanking the RS6904238 polymorphism, the letter N indicates the mutation site corresponding to the T or C nucleotide
**SEQ.3** - shows the nucleotide sequence of a fragment of human genomic DNA flanking the RS743031 polymorphism, the letter N indicates the mutation site corresponding to nucleotide G or A
**SEQ.4** - shows the nucleotide sequence of a fragment of human genomic DNA flanking the RS462904 polymorphism, the letter N indicates the mutation site corresponding to the T or G nucleotide
**SEQ.5** - shows the nucleotide sequence of a fragment of human genomic DNA flanking the RS599721 polymorphism, the letter N indicates the mutation site corresponding to nucleotide G or A
**SEQ.6** - shows the nucleotide sequence of a fragment of human genomic DNA flanking the RS927751 polymorphism, the letter N indicates the mutation site corresponding to nucleotide G or A
**SEQ.7** - shows the nucleotide sequence of a fragment of human genomic DNA flanking the RS675996 polymorphism, the letter N indicates the mutation site corresponding to the T or C nucleotide
**SEQ.8** - shows the nucleotide sequence of a fragment of human genomic DNA flanking polymorphism RS10520667, the letter N indicates the mutation site corresponding to nucleotide T or C
**SEQ.9** - shows the nucleotide sequence of a fragment of human genomic DNA flanking the RS599638 polymorphism, the letter N indicates the mutation site corresponding to the T or C nucleotide
**SEQ.10-** shows the nucleotide sequence of a fragment of human genomic DNA flanking the RS552320 polymorphism, the letter N indicates the mutation site corresponding to nucleotide A or G
**SEQ.11** - shows the nucleotide sequence of a fragment of human genomic DNA flanking the RS10418577 polymorphism, the letter N indicates the mutation site corresponding to the T or C nucleotide
**SEQ.12** - shows the nucleotide sequence of a fragment of human genomic DNA flanking the RS975336 polymorphism, the letter N indicates the mutation site corresponding to nucleotide G or A
**SEQ.13** - shows the nucleotide sequence of the forward primer RS2609473_F
**SEQ.14** - shows the reverse starter sequence RS2609473_R
**SEQ.15** - shows the nucleotide sequence of the RS2609473_P_G probe, which has a fluorescently labeled 5' end with FAM dye and a BHQ1 quencher at the 3' end.
**SEQ.16** - shows the nucleotide sequence of the RS2609473_P_T probe, which has a fluorescently labeled 5' end with HEX dye and a BHQ1 quencher at the 3' end.
**SEQ.17** - shows the nucleotide sequence of the forward primer RS6904238_F
**SEQ.18** - shows the nucleotide sequence of reverse primer RS6904238_R
**SEQ.19** - shows the nucleotide sequence of the RS6904238_P_T probe, which has a fluorescently labeled 5' end with FAM dye and a BHQ1 quencher at the 3' end.
**SEQ.20** - shows the nucleotide sequence of the RS6904238_P_C probe, which has a fluorescently labeled 5' end with HEX dye and a BHQ1 quencher at the 3' end.
**SEQ.21** - shows the nucleotide sequence of forward primer RS743031_F
**SEQ.22** - shows the nucleotide sequence of reverse primer RS743031_R
**SEQ.23** - shows the nucleotide sequence of the RS743031_P_G probe, which has a fluorescently labeled 5' end with FAM dye and a BHQ1 quencher at the 3' end.
**SEQ.24** - shows the nucleotide sequence of the RS743031_P_A probe, which has a fluorescently labeled 5' end with HEX dye and a BHQ1 quencher at the 3' end,
**SEQ.25** - shows the nucleotide sequence of forward primer RS462904_F
**SEQ.26** - shows the nucleotide sequence of reverse primer RS462904_R
**SEQ.27** - shows the nucleotide sequence of the RS462904_P_T probe, which has a fluorescently labeled 5' end with FAM dye and a BHQ1 quencher at the 3' end,
**SEQ.28** - shows the nucleotide sequence of the RS462904_P_G probe, which has a fluorescently labeled 5' end with HEX dye and a BHQ1 quencher at the 3' end,
**SEQ.29** - shows the nucleotide sequence of forward primer RS599721_F
**SEQ.30** - shows the nucleotide sequence of reverse primer RS599721_R
**SEQ.31** - shows the nucleotide sequence of the RS599721_P_G probe, which has a fluorescently labeled 5' end with FAM dye and a BHQ1 quencher at the 3' end,
**SEQ.32** - shows the nucleotide sequence of the RS599721_P_A probe, which has a fluorescently labeled 5' end with HEX dye and a BHQ1 quencher at the 3' end,
**SEQ.33** - shows the nucleotide sequence of forward primer RS927751_F
**SEQ.34** - shows the nucleotide sequence of reverse primer RS927751_R
**SEQ.35** - shows the nucleotide sequence of the RS927751_P_G probe, which has a fluorescently labeled 5' end with FAM dye and a BHQ1 quencher at the 3' end,
**SEQ.36** - shows the nucleotide sequence of the RS927751_P_A probe, which has a fluorescently labeled 5' end with HEX dye and a BHQ1 quencher at the 3' end,
**SEQ.37** - shows the nucleotide sequence of forward primer RS675996_F
**SEQ.38** - shows the nucleotide sequence of reverse primer RS675996_R
**SEQ.39** - shows the nucleotide sequence of the RS675996_P_T probe, which has a fluorescently labeled 5' end with FAM dye and a BHQ1 quencher at the 3' end,
**SEQ.40** - shows the nucleotide sequence of the RS675996_P_C probe, which has a fluorescently labeled 5' end with HEX dye and a BHQ1 quencher at the 3' end,
**SEQ.41** - shows the nucleotide sequence of forward primer RS10520667_F
**SEQ.42** - shows the nucleotide sequence of reverse primer RS10520667_R
**SEQ.43** - shows the nucleotide sequence of the RS10520667_P_T probe, which has a fluorescently labeled 5' end with FAM dye and a BHQ1 quencher at the 3' end,
**SEQ.44** - shows the nucleotide sequence of the RS10520667_P_C probe, which has a fluorescently labeled 5' end with HEX dye and a BHQ1 quencher at the 3' end,
**SEQ.45** - shows the nucleotide sequence of forward primer RS599638_F
**SEQ.46** - shows the nucleotide sequence of reverse primer RS599638_R
**SEQ.47** - shows the nucleotide sequence of the RS599638_P_T probe, which has a fluorescently labeled 5' end with FAM dye and a BHQ1 quencher at the 3' end,
**SEQ.48** - shows the nucleotide sequence of the RS599638_P_C probe, which has a fluorescently labeled 5' end with HEX dye and a BHQ1 quencher at the 3' end,
**SEQ.49** - shows the nucleotide sequence of the forward primer RS552320_F
**SEQ.50** - shows the nucleotide sequence of reverse primer RS552320_R
**SEQ.51** - shows the nucleotide sequence of the RS552320_P_A probe, which has a fluorescently labeled 5' end with FAM dye and a BHQ1 quencher at the 3' end,
**SEQ.52** - shows the nucleotide sequence of the RS552320_P_G probe, which has a fluorescently labeled 5' end with HEX dye and a BHQ1 quencher at the 3' end,
**SEQ.53** - shows the nucleotide sequence of forward primer RS10418577_F
**SEQ.54** - shows the nucleotide sequence of reverse primer RS10418577_R
**SEQ.55** - shows the nucleotide sequence of the RS10418577_P_A probe, which has a fluorescently labeled 5' end with FAM dye and a BHQ1 quencher at the 3' end,
**SEQ.56** - shows the nucleotide sequence of the RS10418577_P_G probe, which has a fluorescently labeled 5' end with HEX dye and a BHQ1 quencher at the 3' end,
**SEQ.57** - shows the nucleotide sequence of forward primer RS975336_F
**SEQ.58** - shows the nucleotide sequence of reverse primer RS975336_R
**SEQ.59** - shows the nucleotide sequence of the RS975336_P_G probe, which has a fluorescently labeled 5' end with FAM dye and a BHQ1 quencher at the 3' end,
**SEQ.60** - shows the nucleotide sequence of the RS975336_P_A probe, which has a fluorescently labeled 5' end with HEX dye and a BHQ1 quencher at the 3' end.

The invention is illustrated by the following examples of implementation, which do not constitute a limitation of the invention

### Example 1

The object of the invention is to use TaqNeqSSB polymerase to standardize the attachment temperature of primers and TaqMan-type probes in the detection of SNPs numbered RS2609473, RS6904238, RS743031, RS462904, RS599721, RS927751, RS675996, RS10520667, RS599638, RS552320, RS10418577, RS975336 in human genomic DNA. The end result of such normalization is a common temperature-time profile for each of the 12 reactions leading to easier and faster SNP detection. For this purpose, 24 forward and reverse DNA oligonucleotides and 24 TaqMan-type probes labeled with FAM or HEX fluorescent dye at the 5' end and BHQ1 quencher at the 3' end were designed:
- forward oligonucleotide named RS2609473_F for use as a forward primer in a real-time PCR reaction (SEQ13),
- reverse oligonucleotide, named RS2609473_R, for use as a reverse primer in a real-time PCR reaction (SEQ14),
- An oligonucleotide identifying the G allele in the RS2609473 polymorphism named RS2609473_P_G, labeled with FAM dye, for use as a probe in a real-time PCR reaction (SEQ15),
- An oligonucleotide identifying the T allele in the RS2609473 polymorphism named RS2609473_P_T, labeled with a HEX dye, for use as a probe in a real-time PCR (SEQ16) reaction, and other analogous 22 oligonucleotides and 22 probes (SEQ17-SEQ18), which allow detection of each allele present in polymorphisms RS6904238, RS743031, RS462904, RS599721, RS927751, RS675996, RS10520667, RS599638, RS552320, RS10418577, RS975336.

Starter forward (SEQ13, SEQ17, SEQ21, SEQ25, SEQ29, SEQ33, SEQ37, SEQ41, SEQ45, SEQ49, SEQ53, SEQ57) is a starter with a sequence starting at the 5' end relative to the leading strand of the target nucleic acid sequence (SEQ1-SEQ12). Starter reverse (SEQ14, SEQ18, SEQ22, SEQ26, SEQ30,SEQ34,SEQ38, SEQ42, SEQ46, SEQ50, SEQ54, SEQ58) is a primer with a sequence starting from the 3' end relative to the leading strand of the target nucleic acid sequence (SEQ1-SEQ12).

A detectable marker or reporter molecule can be attached to the probe. Typical tracers that can be used for probes (SEQ15, SEQ16, SEQ19, SEQ20, SEQ23, SEQ24, SEQ27, SEQ28, SEQ31, SEQ32, SEQ35, SEQ36, SEQ39, SEQ40, SEQ43, SEQ44, SEQ47, SEQ48, SEQ51, SEQ52, SEQ55, SEQ56, SEQ59, SEQ60) include: radioactive isotopes, enzyme substrates, cofactors, ligands, chemiluminescent or fluorescent agents, haptens and enzymes.

Identification of SNPs in human genomic DNA can be identified either in two independent reactions (the same marker for both probes can be used) or in a single reaction using two different markers for each probe. In both cases, the presence of a signal using a probe specific to a given allele indicates its presence in the polymorphism under analysis, the absence of a signal using a probe specific to a given allele indicates its absence in the polymorphism under analysis.

The key ingredient in the reaction mixture is TaqNeqSSB polymerase, which, thanks to its properties of stabilizing the resulting primer-matrix and probe-matrix duplex, increases the specificity of the system, at the same time enabling the reaction to be carried out efficiently over a wide range of oligonucleotide attachment temperatures (FIG6). The normalization of the attachment temperature in the 12 systems studied allowed the emergence of a common temperature for all systems and a uniform temperature-time profile. The range of tolerated oligonucleotide attachment temperatures for TaqNeqSSB polymerase and Taq polymerase without fusion is shown in FIG7. The results indicate that in the case of fusion-free (Taq) polymerase, it is impossible to finetune a common temperature for each system. TaqNeqSSB fusion polymerase allows amplification of all 12 molecular targets at a single temperature - in the example above, it is 63°C.

### Example 2

The example shown above uses TaqNeqSSB polymerase to amplify DNA in a real-time PCR reaction with TaqMan probes. TaqNeqSSB polymerase used in the temeprature normalization of many systems identifying SNP polymorphism or other molecular target can be used in any method of amplification of target DNA fragments such as: polymerase chain reaction (PCR), real-time PCR (qPCR), reverse transcriptase polymerase chain reaction (RT-PCR), real-time reverse transcriptase polymerase chain reaction (RT-qPCR), ligase chain reaction or transcription-mediated amplification (TMA).

TaqNeqSSB polymerase's high sensitivity, inhibitor resistance and tolerance to a wide range of salts (FIG3, FIG4) make it suitable for identification directly from biological samples bypassing the isolation step. Samples can be analyzed directly from buccal swabs, saliva or other clinical specimens after being suspended in saline or other transport buffer of appropriate composition. The result of SNP RS2609473 polymorphism identification using oligomers (SEQ13-SEQ16) for samples identified directly from saliva (bypassing the genetic material isolation step) is shown in FIG8.

Human genomic DNA material is swabs, saliva, tissues or other clinical samples containing human genomic DNA.

### iterature:

Vieille C, Burdette DS, Zeikus JG. Thermozymes. Biotechnol Annu Rev. 1996;2:1-83.
Hamilton SC, Farchaus JW, Davis MC. DNA polymerases as engines for biotechnology. Biotechniques. 2001;31(2):370-6, 378-80, 382-3.
Chien A, Edgar DB, Trela JM. Deoxyribonucleic acid polymerase from the extreme thermophile Thermus aquaticus. J Bacteriol. 1976;127(3):1550-7.
Vainshtein I, Atrazhev a, Eom SH, Elliott JF, Wishart DS, Malcolm B. Peptide rescue of an N-terminal truncation of the Stoffel fragment of Taq DNA polymerase. Protein Sci. 1996;5(9):1785-92.
Barnes WM. The fidelity of Taq polymerase catalyzing PCR is improved by an N-terminal deletion. Gene. 1992;112(1):29-35.
Rittié L, Perbal B. Enzymes used in molecular biology: a useful guide. J Cell Commun Signal. 2008;2(1-2):25-45.
Olszewski M, Balsewicz J, Nowak M, Maciejewska N, Cyranka-Czaja A, Zalewska-Piatek B, Piatek R, Kur J. Characterization of a Single-Stranded DNA-Binding-Like Protein from Nanoarchaeum equitans-A Nucleic Acid Binding Protein with Broad Substrate Specificity. PLoS One. 2015;10(5):e0126563.
Datta K, LiCata VJ. Thermodynamics of the binding of Thermus aquaticus DNA polymerase to primed-template DNA. Nucleic Acids Res. 2003;31(19):5590-7.
Kozlov AG, Lohman TM. Large contributions of coupled protonation equilibria to the observed enthalpy and heat capacity changes for ssDNA binding to Escherichia coli SSB protein. Proteins. 2000;Suppl 4:8-22.

**ID** SEQ1 
AATAAGTACATCTTATTAACTGATTTGACCTGAGGCACTTTGAGGGGTCCTGTTGTAATCTTGCCACTTTCCTCT
GCTTTTCTCATTTATCCCTCTTTTTTTGCAACTTGAGAAGTAAAACAGCAGCCTACCAAGGTGAGGCCACAGTTG
CAGNGATAACATTTGAGCCAAGGCTTCATAAGGGCTGCTGTAGGAGGGGAGAGCATCCTGGCACTTTAGTGG
GCTACGTGGCCGACTGCAATTGCAAGGATTAGCAGTGTGAGCCCTGATCCTGCTA
**ID** SEQ2 
TAGATGGCAGTCACCTTAGCAATAAGCCATCCTGGAAGTGAATCCTCTAACCTCAGTCCCTCCAGTTGCTGTCA
CACATANTAGAGATGAAGCCTTCAGCCAAGTCCTGTCCAAGTTCCTGACCTATAAAATCTTGAGCAAAATAGA
GTACTGTAGTTATTTTAAATCATTCAGCTTTGGGTTAGCTTGTTATGCAACAACAATAATCAAAACACAGGGT
**ID** SEQ3 
GCTTTGGATTCCAGCTCAAGGAGGGTCTGCAGGTTACAGTGAGGGCCCGAAACTCAGAAAGCGAGGGGGCCT
GCCCAGCAGTCTGGCCCGCGGAGCCCTCCCAGCTGCCCATGGATTGCATCACTGTCCCAAGGGGAGACAGGG
CTGTCCTGCCGGCTGCCTCTTTGAGTCACCNTGTGGCCAGGGAGTCCTGCTGGCCTCCACAGTCACCGCCAAAC
CATTTTGGCCTTGGTCTTTCAAAGCCAGGTCATTTTGGGAAAACTGAGGACATCCAGCACCATGGAGGGGCGT
GTGTGTCTCTGCACTGGCTGATGAAGGGAGGTGGG
**ID SEQ4** 
CTAAGTGAACACTGCCTTCCCATTGATGACCTTGAGAGGTTAGAGACCAATGGCAGTGACGGTGATCACATTA
TTATTTGAAGCCTTATTTGAATATTTAAGACTTAGTATGTTTGTTTAAAAAAATAATCATGTTGCATCATAACCAT
ACTTTGTAAGACCAGATTACCGGTGACCGCTCGNGATGTTAAGCTAGTGAAGACAATGCAAGGTGGCCTGAA
GCAGCATGAGGCAGACCATGGTCCCGACCTGCATTGGCTGGTGCTGGCTGCAGCTGCTGATGGAGGCTACAA
ACAGTGATAGAAGCATGCAGAAGCTACACAGCACAGGCCAAGGTGCTGTGGGAGAGGCGGAGGAACAC
TGACCACTGGCGGCCTGGG
**ID** SEQ5 
GGTGCAGACCACAGGCCAGGACTGTGCTAAAGNCTACACTTGGCTTATTGCCCTCAGTCCTCAAAACAGCCCT
GTGCAGTAGGCATTGTAGTTGACCTTGTCTTACAGGTGGGGAGAATGAGCCACAGAGAAAATAAATAATTTCT
GGGAAATCATCCAACAAGCAAATGGTATTGCTGGGATTTTGAGTCCATCCTGCCACCAGCTTCCAGGC
**ID** SEQ6 
TGTTACTACCATAATGGCTTATGTGGTTTATAATGCCTCTTAAATGAACACAATATCAGTCTGCAACCAGGCAG
CAAGGTTTGGGATGANATCATTGTCACAGGGTAGAGAGGCTGCTGGAATCCAACTCATGGCTCAACTGGACTA
ACAACACACACCAGAAACATTCTTACCTTCTGCCTCATGCTTCAGAGAGCTTTAAGCACCAGGTGTAGGTGTGA
TAAGGAATGCGATTAGTGGTATTTATTAAATGAAATATTGTCTATGGAT
**ID** SEQ7 
AGAGAACAGAGGATTCCCTTCCAGGGAAACACACCAACATAAAAGGAAAAACAAAAACAAACAAAAAACTAC
CAATACTAATAGGNGATTGCTGTATTCCAGAGTTCCAGATCCCTGCCTAATCAACCTACAATGAAGTTTCTATTC
AGCCAGGTCAGACCATGTGCACAGAGCTTCCAGTGATCACTTTAGTGCCTCACTCTTAAATGTGACCAGACAAC
CTGGGATCACCAAACATTTGAAGAAATCCTCTAACATGATTAATGATTA
**ID SEQ8** 
ATGTCTTATATCTCTCTTTCTCATCCCAGAGGCAATCGCTCTATTGAAATTTGTTTTCATTGTTATTGTCCTTTTTA
CAAATACCTCTANACTTTCCCACAATCCACACACAGTATTGTTTTACATGTTTTGTAAATGTGCATAAATGACAT
GTCCTTGGTGTCAATTCAGATTTCCTTTTGGGTCAGTACTTTTGTGAAATTCATACATATCGAGGCTTATAGATC
AATTTCATATACTATCATTACTGTTAAATCCAGCATCTGCATA
**ID SEQ9** 
TAAAAGGAAAGTTACCAAAATAATCACATAGCAGATCATCAACCCAATACATAACCCTAGAAATGAAAATTGCC
CCAGCTGTGCCTAT/CCATCCAGCATGTTCCAGTCCTATACTCCTACATCTCTTATTTCTCCACTTTTTTCACGGTT
CTCTGCTTAAATGTGCACACACTGGAGAGGACCTTTTTGAATACCTTAAATGAACTTGTTGTACAAATGTA
**ID SEQ10** 
CCATGTCTACATGTCGCCTAAAGTCATGTCACACACCACATTCATCCCACACTTTGGGAAACTCGCAGCACCAT
GTCATCACTTGCTNAATAGAATCCAGTCGTGGCTTTTCAGGGGACACTGAGACACGCAAAACCTGTCAGGCTG
ATGAGCCAAGGACGTTTCCAGTGGTTTCGCAAGCCTCCTCCAAGTGTTGATGGCTGCCAGCTTTCCTCATCTGG
CCTCTCGTAGCAAATTAACTTAGTCAACATTCACTTACACAAATATTTG
**ID SEQ11** 
GAAGGATGCTGCTTTTGAATGAAACCCAGAGCAAGAGATACCTCTAGAGCAGATTCAGGCTGCAATACAATCC
CCACTGCCAGNGAGTCACATGACAGAGGACTGGAGGTATCTGTAGCAATCAAGATGTGGTTGCATCTTAGGC
AAGTCCTAACAGAAGAGTCTCAGAGAATAACCCTGGGGTTTTGCAGCAAGTCCGTGTCCTCTTGTGTTAGTCTG
CTGGGGCTGCTATTACAAAATACCACAGACTGGGTATTAAACAACAAAACA
**ID SEQ12** 
CAGTAGAGGCGGCATTTGAGGCGCTGACCCATGACAGGAGGAGCACGGGCTACAGCCGAGGACACACTGCT
AGCAGCCATAACNGTGGCAAGTGTTGAACAAGCAGAAAAGATGTAATAGTTGTGAAGTCAGAGGAGTAGTCA
GCGACATTGGCCTGGGTCCTTCCGTGAGGAGCAGACTCGAATGCTAAAATACACTCCCATGCCTCTTCCCACAG
CACTGCCACCACCTAGAATTAAATTTTTGACAGTAACAGCAATCGCGTGTTTT
**ID SEQ13**
   CATGGTATCAGACGCTGGCTC
**ID SEQ14**
   CATGGTATCAGACGCTGGCTC
**ID SEQ15**
   GTGCCTCAGGTCAAATCAGTT
**ID SEQ16**
   CTTGCTTTGCAGGTTGCACGAGACCTA
**ID SEQ17**
   ACTTGCTTTGCAGTTTGCACGAGACCTA
**ID SEQ18**
   AGG AGG ACA CAG AGA GAC CAA
**ID SEQ19**
   AACTTGGACAGGACTTGGCTG
**ID SEQ20**
   CCAGTTGCTGTCACACATATTAGAGATGAAGC
**ID SEQ21**
   CCAGTTGCTGTCACACATACTAGAGATGAAGC
**ID SEQ22**
   AAATGGTTTGGCGGTGACTGT
**ID SEQ23**
   CCACACGGTGACTCAAAGAGGCAGC
**ID SEQ24**
   CCACATGGTGACTCAAAGAGGCAGC
**ID SEQ25**
   TGTTGCATCATAACCATACTTTGT
**ID SEQ26**
   CTTCAGGCCACCTTGCATT
**ID SEQ27**
   CTTCACTAGCTTAACATCACGAGCGGTCAC
**ID SEQ28**
   CTTCACTAGCTTAACATCCCGAGCGGTCAC
**ID SEQ29**
   CAGCTCCCAGTTCATACCAGG
**ID SEQ30**
   CTGTTTTGAGGACTGAGGGCA
**ID SEQ31**
   CCAGGACTGTGCTAAAGGCTACACT
**ID SEQ32**
   CCAGGACTGTGCTAAAGACTACACT
**ID SEQ33**
   AACACAATATCAGTCTGCAACCA
**ID SEQ34**
   TCCAGTTGAGCCATGAGTTGG
**ID SEQ35**
   CCTCTCTACCCTGTGACAATGATCTCATC
**ID SEQ36**
   CCTCTCTACCCTGTGACAATGATTTCATC
**ID SEQ37**
   TCAGGCAAGAGAACAGAGGAT
**ID SEQ38**
   AACTTCATTGTAGGTTGATTAGGC
**ID SEQ39**
   ATCTGGAACTCTGGAATACAGCAATCACCTAT
**ID SEQ40**
   ATCTGGAACTCTGGAATACAGCAATCGCCTAT
**ID SEQ41**
   CCAGAGGCAATCGCTCTATTG
**ID SEQ42**
   AATCTGAATTGACACCAAGGACATG
**ID SEQ43**
   TACCTCTATACTTTCCCACAATCCACACACAGT
**ID SEQ44**
   TACCTCTACACTTTCCCACAATCCACACACAGT
**ID SEQ45**
   TCACATAGCAGATCATCAACCC
**ID SEQ46**
   AAAGGTCCTCTCCAGTGTGTG
**ID SEQ47**
   TGTGCCTATCATCCAGCATGTTCCAGTC
**ID SEQ48**
   TGTGCCTACCATCCAGCATGTTCCAGTC
**ID SEQ49**
   GTCGCCTAAAGTCATGTCACAC
**ID SEQ50**
   TGGAAACGTCCTTGGCTCATCA
**ID SEQ51**
   ATGTCATCACTTGCTAAATAGAATCCAGTCGTGG
**ID SEQ52**
   ATGTCATCACTTGCTGAATAGAATCCAGTCGTGG
**ID SEQ53**
   GCTGCTTTTGAATGAAACCCA
**ID SEQ54**
   ACCACATCTTGATTGCTACAGA
**ID SEQ55**
   TCCTCTGTCATGTGACTCACTGGCAG
**ID SEQ56**
   TCCTCTGTCATGTGACTCGCTGGCAG
**ID SEQ57**
   GTCAGTAGAGGCGGCATTTG
**ID SEQ58**
   TCGCTGACTACTCCTCTGAC
**ID SEQ59**
   CCATAACGGTGGCAAGTGTTGAACAAGC
**ID SEQ60**
   CCATAACAGTGGCAAGTGTTGAACAAGC

## Claims

1. TaqNeqSSB fusion polymerase for use in normalizing the attachment temperature of primers and probes of independent systems identifying several different single-nucleotide mutations in genomic DNA using the example of 12 qPCR systems with TaqMan probes identifying 12 SNP polymorphisms in human genomic DNA in a single temperature-time profile.

2. The use of fusion polymerase according to claim 1, **characterized in that** the standardization applies to systems identifying such SNP polymorphisms as RS2609473, RS6904238, RS743031, RS462904, RS599721, RS927751, RS675996, RS10520667, RS599638, RS552320, RS10418577, RS975336.

3. The use of fusion polymerase according to claim 1, **characterized in that** the sequences of gene fragments containing the above polymorphisms are shown in SEQ1-SEQ12, respectively.

4. The use of fusion polymerase according to claim 1 or 2, **characterized in that** the systems contain:
- The nucleotide sequence of forward primer RS2609473_F shown on SEQ13,
- The nucleotide sequence of reverse primer RS2609473_R shown on SEQ14,
- The nucleotide sequence of probe RS2609473_P_G , which has the sequence shown in SEQ15,
- The nucleotide sequence of probe RS2609473_P_T , which has the sequence shown on SEQ16,
- The nucleotide sequence of forward primer RS6904238_F shown on SEQ17,
- The nucleotide sequence of reverse primer RS6904238_R shown on SEQ18,
- The nucleotide sequence of probe RS6904238_P_T , which has the sequence shown in SEQ19,
- The nucleotide sequence of probe RS6904238_P_C , which has the sequence shown on SEQ20,
- The nucleotide sequence of forward primer RS743031_F shown on SEQ21,
- The nucleotide sequence of reverse primer RS743031_R shown on SEQ22,
- The nucleotide sequence of the probe RS743031_P_G , which has the sequence shown on SEQ23,
- The nucleotide sequence of the probe RS743031_P_A , which has the sequence shown on SEQ24,
- The nucleotide sequence of forward primer RS462904_F shown on SEQ25,
- The nucleotide sequence of reverse primer RS462904_R shown on SEQ26,
- The nucleotide sequence of probe RS462904_P_T, which has the sequence shown on SEQ27,
- The nucleotide sequence of probe RS462904_P_G , which has the sequence shown on SEQ28,
- The nucleotide sequence of forward primer RS599721_F shown on SEQ29,
- The nucleotide sequence of reverse primer RS599721_R shown on SEQ30,
- The nucleotide sequence of the RS599721_P_G probe, which has the sequence shown in SEQ31,
- The nucleotide sequence of the RS599721_P_A probe, which has the sequence shown in SEQ32,
- The nucleotide sequence of forward primer RS927751_F shown on SEQ33,
- The nucleotide sequence of reverse primer RS927751_R shown on SEQ34,
- The nucleotide sequence of probe RS927751_P_G, which has the sequence shown on SEQ35,
- The nucleotide sequence of probe RS927751_P_A, which has the sequence shown on SEQ36,
- The nucleotide sequence of forward primer RS675996_F shown on SEQ37,
- The nucleotide sequence of reverse primer RS675996_R shown on SEQ38,
- The nucleotide sequence of probe RS675996_P_T, which has the sequence shown on SEQ39,
- The nucleotide sequence of the RS675996_P_C probe, which has the sequence shown on SEQ40,
- The nucleotide sequence of forward primer RS10520667_F shown on SEQ41,
- The nucleotide sequence of reverse primer RS10520667_R shown on SEQ42,
- The nucleotide sequence of probe RS10520667_P_T, which has the sequence shown on SEQ43,
- The nucleotide sequence of the RS10520667_P_C probe, which has the sequence shown on SEQ44,
- The nucleotide sequence of forward primer RS599638_F shown on SEQ45,
- The nucleotide sequence of reverse primer RS599638_R shown on SEQ46,
- The nucleotide sequence of the RS599638_P_T probe, which has the sequence shown on SEQ47,
- The nucleotide sequence of the RS599638_P_C probe, which has the sequence shown on SEQ48,
- The nucleotide sequence of forward primer RS552320_F shown on SEQ49,
- The nucleotide sequence of reverse primer RS552320_R shown on SEQ50,
- The nucleotide sequence of the RS552320_P_A probe, which has the sequence shown on SEQ51,
- The nucleotide sequence of the RS552320_P_G probe, which has the sequence shown on SEQ52,
- The nucleotide sequence of forward primer RS10418577_F shown on SEQ53,
- The nucleotide sequence of reverse primer RS10418577_R shown on SEQ54,
- The nucleotide sequence of probe RS10418577_P_A, which has the sequence shown on SEQ55,
- The nucleotide sequence of probe RS10418577_P_G, which has the sequence shown on SEQ56,
- The nucleotide sequence of forward primer RS975336_F shown on SEQ57,
- The nucleotide sequence of reverse primer RS975336_R shown on SEQ58,
- The nucleotide sequence of the RS975336_P_G probe, which has the sequence shown on SEQ59,
- The nucleotide sequence of the RS975336_P_A probe, which has the sequence shown on SEQ60.

5. The primers and probes defined above SEQ.13-SEQ.60 have different attachment temperatures for molecular targets and are specific for the 12 SNP polymorphic sequences listed earlier: RS2609473, RS6904238, RS743031, RS462904, RS599721, RS927751, RS675996, RS10520667, RS599638, RS552320, RS10418577, RS975336.

6. A method of normalizing the attachment temperature of primers and probes of multiple systems to a single optimal one, using the polymorphisms defined in claim 5 as an example, comprising the steps:
1)DNA amplification using primers and probes with SEQ.13 - SEQ.60 in a temperature gradient
2) Detection of the formed complexes by observing the increase in fluorescence of the dye with which the probe is labeled.
3) Selection of a common temperature for all systems, at which Ct is as low as possible and similar for each system

7. The method according to claim 6, **characterized in that** the human gene material is a swab, saliva, blood, tissues or other biological samples containing human genomic DNA.

8. The method according to claim 6, **characterized in that** step 1) uses TaqNeqSSB polymerase.

9. The method according to claim 6, **characterized by** the fact that in step 2) labeled probes are used.

10. A kit for use in the manner defined in claim 6, comprising:
Starters
Probes
TaqNeqSSB DNA polymerase with cofactor
Deoxyribonucleoside triphosphates
Reaction buffer

11. The kit according to claim 10, **characterized by** the fact that it contains reagents required in the amplification steps: denaturation, hybridization, DNA strand synthesis.

12. Primers and probes containing SEQ.13 - SEQ.60 for use in detecting and identifying single-nucleotide mutation numbers RS2609473, RS6904238, RS743031, RS462904, RS599721, RS927751, RS675996, RS10520667, RS599638, RS552320, RS10418577, RS975336.
